# EUROPEAN PATENT APPLICATION

(11) **EP 4 614 152 A1**
(43) Date of publication of application: **10.09.2025**
(21) Application number: 23885761.9
(22) Date of filing: 31.10.2023
(51) Int. Cl.: G01N 33/66, A61B 5/154, A61B 5/157, G01N 33/48

(54) **INHIBITOR FOR GLUCOSE UPTAKE INTO RED BLOOD CELLS, SUPPRESSANT FOR GLUCOSE CONCENTRATION REDUCTION IN BLOOD-COLLECTING TUBE, AND BLOOD-COLLECTING TUBE INCLUDING SAME**

(30) Priority: 31.10.2022 JP 2022174451
(71) Applicant: The University of Tokyo, Bunkyo-ku, Tokyo 113-8654 (JP)
(72) Inventor: KUME, Yukio, Tokyo 113-8654 (JP); KURANO, Makoto, Tokyo 113-8654 (JP)
(74) Representative: Regimbeau
(86) International application number: PCT/JP2023/039256
(87) International publication number: WO 2024/096007

(57) **Abstract**

An object of the present invention is to provide an agent for inhibiting glucose uptake into red blood cells and a blood collection tube that suppresses glucose concentration decrease. According to the present invention, there is provided an agent for inhibiting glucose uptake into red blood cells, including inosine as an active ingredient. The inhibition agent of the present invention can be used for suppressing a decrease in glucose concentration of whole blood collected in a blood collection tube. According to the present invention, there is also provided a blood collection tube including inosine in an internal space. The blood collection tube of the present invention can be used to measure a glucose concentration in blood. The blood collection tube of the present invention can further include a glycolysis inhibitor.

## Description

### CROSS-REFERENCE TO RELATED APPLICATION

The present application enjoys the benefit of priority from the prior Japanese Patent Application No. 2022-174451 filed on October 31, 2022, the entire disclosures of which are incorporated herein by reference.

### TECHNICAL FIELD

The present invention relates to an agent for inhibiting glucose uptake into red blood cells. The present invention also relates to an agent for suppressing glucose concentration decrease in a blood collection tube and a blood collection tube including an agent for suppressing glucose concentration decrease.

### BACKGROUND ART

The blood glucose level is an essential item of diagnostic criteria for diabetes and gestational diabetes (glucose 126 mg/dL or more), and extremely high precision and accuracy are required for the measurement thereof. An allowable variation range of the blood glucose level defined by the Japanese Society of Clinical Chemistry is ±4 mg/dL or less. When collected blood is stored at room temperature in a blood collection tube in a state of whole blood, a glucose concentration may decrease. This is because glucose is taken up by red blood cells in blood, glucose is broken down by glycolytic enzymes within the red blood cells, and the glucose concentration in the blood decreases over time.

After blood collection, the blood is immediately centrifuged to separate the red blood cells and the supernatant, and a blood glucose level is measured in the supernatant, whereby a decrease in a glucose concentration as described above can be avoided. However, in a case where blood is collected at a private clinic or a medical examination venue, it often takes up to about 12 hours to arrive at a clinical examination facility after the blood collection. Most private practitioner's clinics and medical examination venues are not equipped with a centrifuge, and therefore, in a case where blood is collected in these facilities, the blood is stored in a blood collection tube for about 12 hours at maximum from the blood collection to the centrifugation in the state of whole blood after the blood collection, and a decrease in glucose concentration cannot be avoided. For this reason, in a case where blood is collected at a private clinic or a medical examination venue, there is a possibility that diabetes will be overlooked.

A blood collection tube having sodium fluoride in the tube as a glycolysis inhibitor has been put into practical use, and furthermore, a blood collection tube having adenosine triphosphate (ATP) in the tube as a glycolysis inhibitor has also been proposed (Patent Document 1).

On the other hand, since sodium fluoride is insufficient to suppress a time-dependent decrease in a glucose concentration for the first 4 hours, as a countermeasure, the guidelines of the Clinical and Biochemistry Academy of the United States recommend that blood be immersed in an ice-water slurry immediately after collection and that in a case where plasma separation cannot be performed within 30 minutes, a citrate buffer solution be used (Non Patent Document 1). However, a blood collection tube containing a citrate buffer solution cannot be used to measure HbA1c, and has not been used much in Japan from the viewpoint of efficiency and economy.

### Reference List

### Patent Documents

Patent Document 1: JP 2019-2821 A

### Non Patent Document

Non Patent Document 1: David B. Sacks et al., Guidelines and Recommendations for Laboratory Analysis in the Diagnosis and Management of Diabetes Mellitus, Clinical Chemistry 57(6); e1-e7(2011)

### SUMMARY OF THE INVENTION

An object of the present invention is to provide an agent for inhibiting glucose uptake into red blood cells. Another object of the present invention is to provide an agent for suppressing glucose concentration decrease in a blood collection tube. Still another object of the present invention is to provide a blood collection tube that suppresses a decrease in glucose concentration of collected blood.

The present inventors have found that inosine inhibits glucose uptake into red blood cells, that a decrease in glucose concentration in whole blood is avoided by adding inosine to a blood collection tube, and that HbA1c, insulin, and C-peptide can be measured using a blood collection tube to which inosine has been added. The present invention is based on these findings.

According to the present invention, the following inventions are provided.
[1] An agent for inhibiting glucose uptake into red blood cells, comprising inosine as an active ingredient.
[2] An agent for suppressing glucose concentration decrease in a blood collection tube, comprising inosine as an active ingredient.
[3] The agent for suppressing glucose concentration decrease according to [2], further comprising a glycolysis inhibitor.
[4] The agent for suppressing glucose concentration decrease according to [3], wherein the glycolysis inhibitor is one or more selected from the group consisting of a fluoride salt and adenosine phosphate or a salt thereof.
[5] The agent for suppressing glucose concentration decrease according to any one of [2] to [4], further comprising an anticoagulant.
[6] The agent for suppressing glucose concentration decrease according to [5], wherein the anticoagulant is one or more selected from the group consisting of ethylenediaminetetraacetic acid (EDTA) and salts thereof, and hydrates thereof, citric acid and a salt thereof, and heparin and a salt thereof.
[7] The agent for suppressing glucose concentration decrease according to any one of [2] to [6], wherein the inosine is used at 0.10 mg or more per 1 mL of collected blood.
[8] A blood collection tube for measuring a glucose concentration in blood, comprising the agent for suppressing glucose concentration decrease according to any one of [2] to [7] in an internal space.
[9] The blood collection tube according to [8], wherein the agent for suppressing glucose concentration decrease contains 0.10 mg or more of inosine per 1 mL of collected blood.
[10] The blood collection tube according to [8] or [9], for further measuring one or more selected from the group consisting of HbA1c, insulin, and C-peptide.
[11] A method for inhibiting glucose uptake into red blood cells in whole blood or a method for suppressing a decrease in a glucose concentration in whole blood, comprising mixing whole blood and inosine in vitro.
[12] The method according to [11], further comprising mixing a glycolysis inhibitor and/or an anticoagulant with whole blood.
[13] The method according to [11] or [12], wherein the inosine is mixed in at 0.10 mg or more per 1 mL of whole blood.
[14] The method according to [11] or [12], which is performed in a blood collection tube.

According to the present invention, it is possible to provide a blood collection tube that can avoid a decrease in glucose concentration even when whole blood is stored in a blood collection tube for a long time. When the glucose concentration is measured using a conventional blood collection tube containing sodium fluoride and ethylenediaminetetraacetic acid (EDTA) in a tube, it can be observed that the glucose concentration in the blood tends to decrease even after about 4 hours, and therefore, for example, even in the case of diabetes, a negative determination may be made (false negative). According to the present invention, it is possible to accurately measure a blood glucose level regardless of a facility where blood is collected, which is advantageous in terms of contributing to early detection of diabetes and curbing diabetes being overlooked.

### BRIEF DESCRIPTION OF THE DRAWINGS

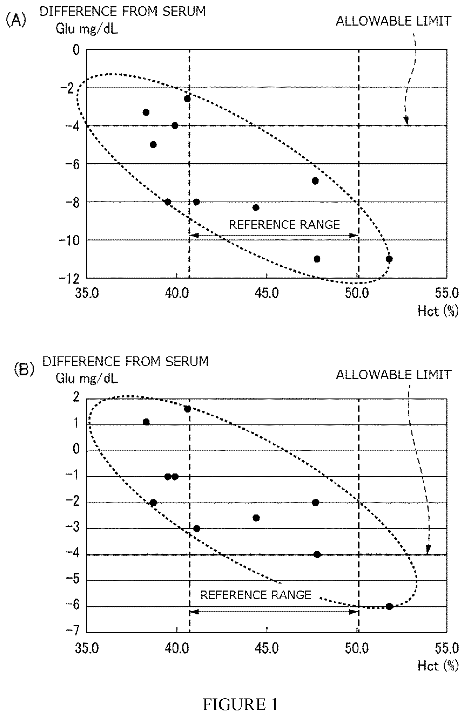

Fig. 1 is a view showing a relationship between a glucose concentration change and a hematocrit value after 12 hours of whole blood being stored in a blood collection tube. (A) shows a case where a blood collection tube C is used, and (B) shows a case where a blood collection tube K is used.

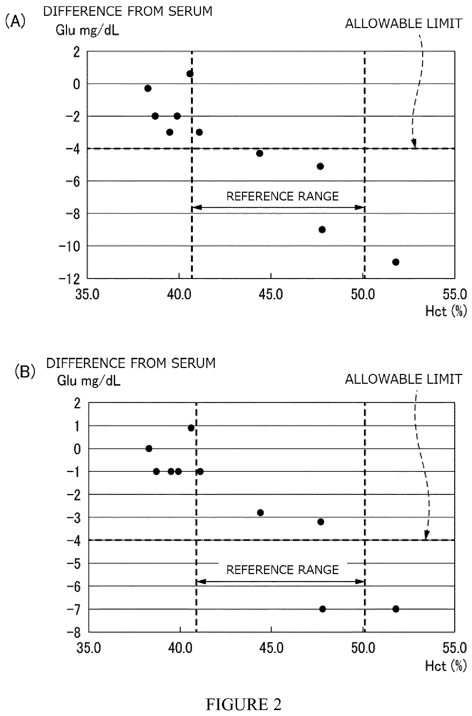

Fig. 2 is a view showing a relationship between a glucose concentration change and a hematocrit value after 12 hours in whole blood being stored in a blood collection tube. (A) shows a case where a blood collection tube I is used, and (B) shows a case where a blood collection tube J is used.

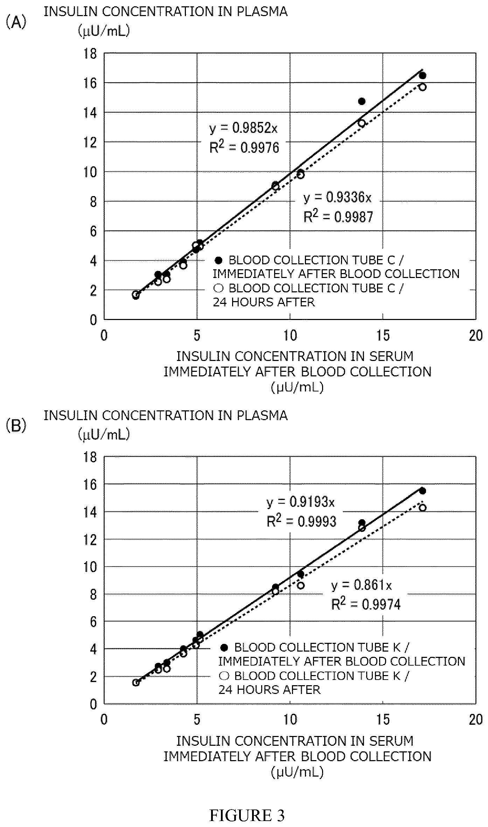

Fig. 3 is a view showing a change in an insulin concentration after 24 hours in whole blood being stored in a blood collection tube. (A) shows a case where a conventional blood collection tube (blood collection tube C) is used, and (B) shows a case where a blood collection tube (blood collection tube K) of the present invention to which inosine has been added is used.

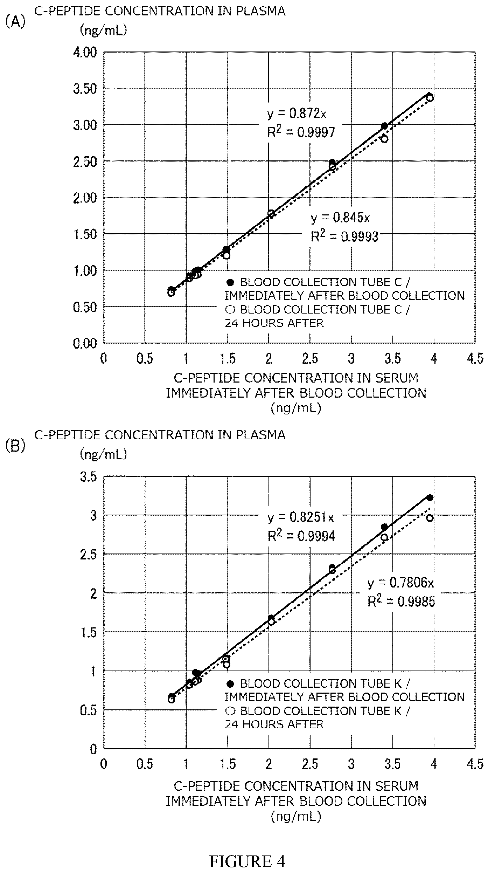

Fig. 4 is a view showing a change in a c-peptide concentration after 24 hours in whole blood being stored in a blood collection tube. (A) shows a case where a conventional blood collection tube (blood collection tube C) is used, and (B) shows a case where a blood collection tube (blood collection tube K) of the present invention to which inosine has been added is used.

### DETAILED DESCRIPTION OF THE INVENTION

### <<Agent for inhibiting glucose uptake into red blood cells>>

According to the present invention, there is provided an agent for inhibiting glucose uptake into red blood cells, containing inosine as an active ingredient. Glucose transporter protein (GLUT1) is present on a cell membrane of a red blood cell, and is responsible for the uptake of glucose into red blood cells. It is considered that the inhibition agent of the present invention acts on GLUT1 and inhibits glucose uptake by GLUT1. That is, according to the present invention, an agent for inhibiting glucose transport by GLUT1 is provided.

According to another aspect of the present invention, a method for inhibiting glucose uptake into red blood cells in whole blood, including mixing whole blood and inosine in vitro is provided. In addition to inosine, a glycolysis inhibitor and/or an anticoagulant may be mixed into whole blood. Mixing whole blood and inosine includes a mode in which whole blood is collected in a blood collection tube provided with inosine therein, and then mixed by inversion. That is, the method for inhibiting glucose uptake of the present invention can be carried out in a blood collection tube. The method for inhibiting glucose uptake of the present invention can be carried out according to the description of the inhibition agent of the present invention.

### <<Agent for suppressing glucose concentration decrease in blood collection tube>>

The inhibition agent of the present invention can inhibit glucose uptake into red blood cells, and therefore, can prevent the breakdown of glucose by glycolytic enzymes present in red blood cells. Therefore, the inhibition agent of the present invention can be used to suppress a decrease in a glucose concentration of whole blood being stored in the blood collection tube. That is, according to the present invention, there is provided an agent for suppressing glucose concentration decrease in a blood collection tube including inosine as an active ingredient.

A lower limit of the amount of inosine used per 1 mL of collected blood can be 0.10 mg, 0.125 mg, 0.25 mg, 0.375 mg, 0.50 mg, 0.75 mg, 1.0 mg, 1.25 mg, or 1.5 mg, and an upper limit of the amount of inosine used per 1 mL of collected blood can be 5.0 mg, 4.0 mg, or 3.0 mg. The range of the amount of inosine used per 1 mL of collected blood can be set by combining the above lower limit and upper limit, and can be, for example, 0.10 to 5.0 mg, 0.125 to 5.0 mg, 0.10 to 4.0 mg, or 0.125 to 4.0 mg.

Inosine can be used singly in the agent for suppressing glucose concentration decrease of the present invention, and can also be used in combination with a glycolysis inhibitor. Examples of the glycolysis inhibitor include a fluoride salt, adenosine phosphate or a salt thereof. When inosine is used in combination with a glycolysis inhibitor (particularly, a fluoride salt), the effect of suppressing glucose concentration decrease can be more strongly exhibited.

The fluoride salt is not particularly limited, and examples thereof include sodium fluoride and potassium fluoride, and sodium fluoride is preferable. These can be used singly or in combination of two or more kinds thereof. Sodium fluoride inhibits enolase in a glycolytic pathway, thereby exerting an effect of inhibiting glucose breakdown in a blood collection tube.

In a case where the fluoride salt is used as an agent for suppressing glucose concentration decrease in a blood collection tube, it is preferable to use the fluoride salt in the range of 0.2 to 3.0 mg, more preferably in the range of 1.0 to 2.5 mg, and still more preferably in the range of 1.5 to 2.0 mg per 1 mL of collected blood.

Examples of the adenosine phosphate and a salt thereof of the present invention include adenosine triphosphate (ATP), adenosine diphosphate (ADP), and adenosine monophosphate (AMP), and salts thereof, and ATP and salts thereof are preferable. These can be used singly or in combination of two or more kinds thereof.

Examples of the salt of adenosine phosphate include alkali metal salts such as a sodium salt and a potassium salt, and alkaline earth metal salts such as a calcium salt and a magnesium salt, and an alkali metal salt is preferable, and a sodium salt is more preferable. As the salt of ATP, a disodium salt is suitable.

When adenosine phosphate or a salt thereof is used as the agent for suppressing glucose concentration decrease in a blood collection tube, adenosine phosphate or a salt thereof is preferably used in the range of 0.1 to 1000 mg, more preferably in the range of 0.5 to 200 mg, and still more preferably in the range of 1 to 100 mg per 1 mL of collected blood.

The agent for suppressing glucose concentration decrease of the present invention can be used in combination with an anticoagulant. Examples of the anticoagulant include ethylenediaminetetraacetic acid (EDTA) or a salt thereof or a hydrate thereof, citric acid or a salt thereof (for example, alkali metal salts of citric acid, such as sodium citrate), and heparin or a salt thereof (for example, alkali metal salts of heparin, such as sodium heparin).

The salt of EDTA is not particularly limited, and examples thereof include EDTA alkali metal salts (for example, disodium EDTA and dipotassium EDTA). EDTA and a salt thereof and a hydrate thereof can be used singly or in combination of two or more kinds thereof.

When EDTA or a salt thereof or a hydrate thereof is used as an anticoagulant of blood, EDTA or a salt thereof or a hydrate thereof is preferably used in the range of 0.5 to 2.5 mg as an EDTA anhydrous salt, more preferably in the range of 0.75 to 2.25 mg, and still more preferably in the range of 1 to 2 mg or 1.2 to 2 mg per 1 mL of collected blood.

The agent for suppressing glucose concentration decrease of the present invention is intended for use in a blood collection tube, and therefore may contain a glycolysis inhibitor and/or an anticoagulant in addition to inosine, or may be a combination of inosine and a glycolysis inhibitor and/or an anticoagulant.

According to another aspect of the present invention, a method for suppressing a decrease in a glucose concentration in whole blood, including mixing whole blood and inosine in vitro is provided. In addition to inosine, a glycolysis inhibitor and/or an anticoagulant may be mixed into whole blood. Mixing whole blood and inosine includes a mode in which whole blood is collected in a blood collection tube provided with inosine therein, and then mixed by inversion. That is, the method for suppressing a decrease in a glucose concentration of the present invention can be carried out in a blood collection tube. The method for suppressing a decrease in a glucose concentration of the present invention can be carried out according to the description of the agent for suppressing glucose concentration decrease of the present invention.

### <<Blood collection tube>>

A blood collection tube of the present invention includes a cylindrical body having an upper end having an opening and a lower end having a bottom in a longitudinal direction, and a stopper that closes the opening at the upper end. In addition, the inhibition agent of the present invention or the agent for suppressing glucose concentration decrease of the present invention is contained in an internal space of the cylindrical body. That is, the blood collection tube of the present invention includes at least inosine in the cylindrical internal space.

The upper end having an opening and the lower end having a bottom are both end portions of the cylindrical body in the longitudinal direction, and when the blood collection tube is used, the upper end is positioned higher than the lower end relative to the ground and blood is collected from the opening, and the lower end is positioned lower than the upper end relative to the ground and receives the collected blood with the bottom. Also, the cylindrical body preferably has an annular cross section. When the cross section is annular, the cross section may be circular or substantially circular.

A material of the cylindrical body is not particularly limited, and examples thereof include glass and plastics such as polyethylene terephthalate. In order to make the internal state visible, a colorless and transparent material is preferable.

The stopper for closing the opening at the upper end of the cylindrical body is not particularly limited, and examples thereof include a rubber stopper and a film stopper. The stopper central portion may be set to be thinner than portions other than the stopper central portion so that puncture of a blood sampling needle into the stopper can be easily and safely performed. In addition, in the blood collection tube, it is preferable that the stopper close the opening at the upper end of the cylindrical body and the internal space of the cylindrical body be depressurized. Due to the internal space of the cylindrical body being depressurized, blood collection into the blood collection tube tends to be facilitated. The degree of depressurization can be appropriately set according to the amount of blood collected and the degree of sealing of the internal space of the cylindrical body by the stopper.

In the present invention, the position of the internal space of the cylindrical body containing an additive such as inosine is preferably on a lower end side of the upper end of the cylindrical body since this makes it easier to come into contact with the collected blood.

In addition to the inhibition agent of the present invention or the agent for suppressing glucose concentration decrease of the present invention, the blood collection tube of the present invention may further include a pH adjusting agent for suppressing glycolysis of red blood cells in the collected blood and/or for inhibiting hemolysis (which may occur when the pH is 4 or lower) of the collected blood.

The pH adjusting agent is not particularly limited, and examples thereof include citric acid and succinic acid and salts thereof, and these can be used singly or in combination of two or more kinds thereof depending on the purpose.

In the present invention, the additive such as inosine may be accommodated in the internal space of the blood collection tube in any form such as granules, powder, a sheet, or a tablet, or may be spray-coated on a wall surface of the blood collection tube, dried, and fixed to the wall surface. Two or more kinds of additives such as inosine may be mixed and accommodated in the internal space of the blood collection tube, or may be separately accommodated in the internal space of the blood collection tube.

As shown in Examples described later, when HbA1c was measured for a blood sample collected by the blood collection tube of the present invention, the blood collection tube of the present invention did not affect the measurement result. Further, when insulin and c-peptide were each measured for a blood sample collected by the blood collection tube of the present invention, a certain decrease was observed as compared with the serum immediately after blood collection, but a high correlation was observed in relationship therebetween, and thus it was considered to be sufficiently applicable for clinical use when using a coefficient. That is, since the blood collection tube of the present invention can be used for measurement of all four items of glucose, HbA1c, insulin, and C-peptide, the present invention is an advantageous invention in terms of contributing to improvement of test efficiency and cost reduction.

### EXAMPLES

The present invention will be described more specifically based on the following examples, but the present invention is not limited to these examples.

### Blood collection tube

Commercially available blood collection tubes (Blood collection tubes A, B, C, and D) used in the examples are as follows.

**[Table 1]**

| Table 1: Overview (1) of blood collection tubes used in Examples | | | |
|---|---|---|---|
| Blood collection tube type | Common name | Content 1 | Content 2 |
| Blood collection tube A | For biochemistry: Containing high-speed coagulation and separation agent | Thrombin | Separation agent |
| Blood collection tube B | For complete blood count (EDTA-2k) | EDTA-2k (4.5 mg) | |
| Blood collection tube C | For blood glucose | EDTA-2Na (8.2 mg) | NaF (3.0 mg) |
| Blood collection tube D | For hematology (EDTA-2Na) | EDTA-2Na | |

| | | | |
|---|---|---|---|
| Blood collection tube A: Insepack II-D, blood collection tube containing high-speed coagulation and separation agent for biochemistry and serology, SIM-L1008SQ3 Tokuyama Sekisui Co., Ltd. Blood collection tube B: Nipro Neo-tube A, EDTA-2k (granule), NP-EK0205, Nipro Ltd. Blood collection tube C: Nipro Neo-tube A, sodium fluoride + EDTA-2Na (granule), OP-FN0205, Nipro Ltd. Blood collection tube D: Insepack II, 5 mL blood collection tube for hematology, SMD750ENA, Tokuyama Sekisui Co., Ltd. | | | |

A blood collection tube prepared based on a commercially available blood collection tube is as follows.

**[Table 2]**

| Table 2: Overview (2) of blood collection tubes used in Examples | | | |
|---|---|---|---|
| Blood collection tube type | Base blood collection tube | Additive 1 | Additive 2 |
| Blood collection tube E | Blood collection tube B | Inosine 1.0 mg | |
| Blood collection tube F | Blood collection tube B | Inosine 2.0 mg | |
| Blood collection tube G | Blood collection tube B | Inosine 3.0 mg | |
| Blood collection tube H | Blood collection tube C | ATP 5.0 mg | |
| Blood collection tube I | Blood collection tube C | ATP 15.0 mg | |
| Blood collection tube J | Blood collection tube C | ATP 20.0 mg | |
| Blood collection tube K | Blood collection tube C | ATP 5.0 mg | Inosine 2 mg |
| Blood collection tube L | Blood collection tube C | Inosine 0.75 mg | |
| Blood collection tube M | Blood collection tube C | Inosine 1.50 mg | |
| Blood collection tube N | Blood collection tube C | Inosine 2.25 mg | |
| Blood collection tube O | Blood collection tube C | Inosine 3.00 mg | |
| Blood collection tube P | Blood collection tube C | Inosine 3.75 mg | |
| Blood collection tube Q | Blood collection tube C | Inosine 4.50 mg | |
| Blood collection tube R | Blood collection tube C | ATP 5.0 mg | Inosine 1.50 mg |
| Blood collection tube S | Blood collection tube C | ATP 5.0 mg | Inosine 3.00 mg |
| Blood collection tube T | Polystyrene Spitz | *1 Partial plasma disposal | |
| Blood collection tube U | Blood collection tube C | *1 Partial plasma disposal | |
| Blood collection tube V1 | Blood collection tube C | *1 Partial plasma disposal | Inosine 0.25 |
| Blood collection tube V2 | Blood collection tube C | *1 Partial plasma disposal | Inosine 0.50 |
| Blood collection tube V3 | Blood collection tube C | *1 Partial plasma disposal | Inosine 0.75 |
| Blood collection tube V4 | Blood collection tube C | *1 Partial plasma disposal | Inosine 1.00 |
| Blood collection tube V5 | Blood collection tube C | *1 Partial plasma disposal | Inosine 1.25 |

| | | | |
|---|---|---|---|
| *1: Blood obtained by discarding 0.85 mL of plasma from 5 ml of blood collected by the blood collection tube D was used. | | | |

The blood collection tube of Table 2 was prepared by immersing each additive in a filter paper after dissolving it in a solution, and adding the additive to the blood collection tube in a state of being dried in a refrigerator (4°C) for 48 hours. Specifically, for ATP (FUJIFILM Wako Pure Chemical Corporation, Code No. 309-50513), a 200 mg/mL solution was prepared, 25 µL (corresponding to an ATP amount of 5 mg) thereof was attached to filter paper (Qualitative filter paper No. 131, Advantec Ltd.) cut into a diameter of 6 mm and dried, and the required number of sheets were charged into the blood collection tube. For inosine (FUJIFILM Wako Pure Chemical Corporation, Code No. 099-00231), a 10 mg/mL:10 mg/mL solution was prepared, 25 µL (corresponding to an amount of inosine of 0.25 mg) thereof was attached to filter paper (Qualitative filter paper No. 131, Advantec Ltd.) cut into a diameter of 6 mm, and dried, and the required number of sheets were charged into the blood collection tube.

### Experimental Methods

In the following examples, an automatic glucose measuring device (Adams glucose GA-1153, GOD electrode method, Arkley) was used for measuring blood glucose (glucose concentration).

Regarding serum, the blood was centrifuged at 3000 rpm for 5 minutes within 5 minutes after collection, and the supernatant (serum) was transferred to a separate test tube, which was used as a sample to measure the glucose concentration. Regarding the blood collection tube other than serum, blood cells and supernatant (plasma) were separated from each other by centrifugation at 3000 rpm for 5 minutes, and the separated supernatant (plasma) was used for measurement of glucose concentration.

### Example 1: Effect of inosine on decrease in measurement value of glucose concentration of blood in blood collection tube

### (1) Methods

Blood was collected in the following various blood collection tubes from 10 volunteer subjects, and after the blood collection, the tubes were stored at room temperature for up to 4 hours in a whole blood state (that is, uncentrifuged), and thereafter, the tubes were stored at 4°C for up to 24 hours. The glucose concentration (mg/dL) was measured using the blood collection tubes C, I, J, and K 12 hours after the blood collection, and this measured value was taken as a glucose concentration in a blood collection tube.

In addition, a hematocrit value of the blood in the whole blood state (that is, uncentrifuged) collected in the blood collection tube B (for complete blood count) within 4 hours from the blood collection was measured with a multi-item automatic blood cell analyzer XN-1000 (Sysmex Corporation).

Regarding serum, supernatants (serum) of blood collected from 10 volunteer subjects were stored at room temperature for up to 4 hours, and thereafter stored at 4°C for up to 24 hours. The glucose concentration (mg/dL) was measured up to 24 hours after the blood collection, and this measured value was taken as a serum glucose concentration at each time.

### (2) Results

The numerical values obtained by subtracting the serum glucose concentration from the glucose concentration in a blood collection tube were shown for each subject in Fig. 1(A) (blood collection tube C), Fig. 1(B) (blood collection tube K), Fig. 2(A) (blood collection tube I), and Fig. 2(B) (blood collection tube J). In the conventional blood collection tube C including sodium fluoride as a glycolysis inhibitor, even when the hematocrit value was within the reference range (40.7% to 50.1%), the glucose concentration decreased beyond the allowable measurement variation range (±4 mg/dL) of the blood glucose level due to storage in 4 cases out of 5 cases. In the blood collection tubes I and J to which ATP was added, even when the hematocrit value fell within the reference range (40.7% to 50.1%), 3 cases out of 5 cases (ATP 15 mg) and 1 case out of 5 cases (ATP 20 mg) showed that the glucose concentration decreased beyond the allowable measurement variation range (±4 mg/dL) of the blood glucose level due to storage. On the other hand, in the blood collection tube K in which ATP was reduced to 5 mg and then 2 mg of inosine was added, there was a case where the glucose concentration decreased beyond the allowable measurement variation range (±4 mg/dL) in the case where the hematocrit value exceeded the reference range (40.7% to 50.1%), but there was no case where the glucose concentration exceeded the measurement allowable variation range in the five cases where the hematocrit value was within the reference range.

### Example 2: Effect of various components on glucose concentration decrease in blood collection tube

### (1) Methods

Blood was collected in the following various blood collection tubes from one volunteer subject, and after the blood collection, serum obtained by centrifuging immediately thereafter was used, and other blood was stored at room temperature for up to 4 hours in a whole blood state (that is, uncentrifuged), and thereafter, the tubes were stored at 4°C for up to 24 hours. The glucose concentrations (mg/dL) of the blood collection tubes B, C, H, L, M, N, O, P, Q, R, and S immediately after blood collection and 4 hours, 12 hours, and 24 hours after blood collection were measured, and the measured value was taken as the glucose concentration in a blood collection tube.

Regarding serum, supernatants (serum) of blood collected from one volunteer subject were stored at room temperature for up to 4 hours, and thereafter stored at 4°C for up to 24 hours. As a blood collection tube, polystyrene spits were used. The glucose concentration (mg/dL) was measured up to 4 hours after the blood collection, and this measured value was taken as a serum glucose concentration at each time.

### (2) Results

The glucose concentration of the serum immediately after blood collection and the time-dependent change of the glucose concentration in the serum and various blood collection tubes are shown in Table 3 below.

**[Table 3]**

| Table 3: Time-dependent change of glucose concentration in various blood collection tubes | | | | | | |
|---|---|---|---|---|---|---|
| Blood collection tube | ATP Additive amount (mg) | Inosine Additive amount (mg) | 0 hours (immediately after) (mg/dL) | After 4 hours (mg/dL) | After 12 hours (mg/dL) | After 24 hours (mg/dL) |
| Serum | - | - | 100 | 99 | 99 | 100 |
| Blood collection tube B | - | - | 99 | 87 | 63 | 28 |
| Blood collection tube C | - | - | 100 | 93 | 90 | 90 |
| Blood collection tube H | 5 | - | 100 | 96 | 91 | 90 |
| Blood collection tube L | - | 0.75 | 100 | 97 | 95 | 95 |
| Blood collection tube M | - | 1.50 | 100 | 97 | 95 | 95 |
| Blood collection tube N | - | 2.25 | 100 | 97 | 95 | 95 |
| Blood collection tube O | - | 3.00 | 100 | 97 | 95 | 94 |
| Blood collection tube P | - | 3.75 | 100 | 98 | 95 | 94 |
| Blood collection tube Q | - | 4.50 | 100 | 97 | 95 | 94 |
| Blood collection tube R | 5 | 1.50 | 100 | 99 | 97 | 97 |
| Blood collection tube S | 5 | 3.00 | 100 | 99 | 97 | 97 |

In the conventional blood collection tube C including sodium fluoride as a glycolysis inhibitor and the blood collection tube H prepared by adding 5 mg of ATP to the blood collection tube C, the glucose concentration after 12 hours and the glucose concentration after 24 hours (mg/dL) were 91, 90, 90, and 90, respectively. On the other hand, in the blood collection tubes L, M, N, O, P, and Q prepared by adding inosine to the blood collection tube C, the glucose concentrations (mg/dL) after 24 hours were all 94 or more, and a high glucose concentration decrease suppressing effect was recognized. In addition, in the blood collection tubes R and S prepared by adding inosine and ATP to the blood collection tube C, the glucose concentrations (mg/dL) after 24 hours were both 97, and a higher glucose concentration decrease suppressing effect was recognized. According to the present invention, it was possible to achieve a high effect of suppressing glucose concentration decrease by using inexpensive inosine while not using expensive ATP or reducing the amount of ATP used.

### Example 3: Effect of inosine on glucose concentration decrease in blood collection tubes

### (1) Methods

Blood was collected from one volunteer subject in the following various blood collection tubes (blood collection tubes T, U, and V1 to V5).

Regarding serum, supernatants (serum) of blood collected from one volunteer subject were stored at room temperature for up to 4 hours, and thereafter stored at 4°C for up to 24 hours. As a blood collection tube, polystyrene spits were used. The glucose concentration (mg/dL) was measured up to 4 hours after the blood collection, and this measured value was taken as a serum glucose concentration at each time.

For the blood collection tube T, 5 mL of blood was collected in each of the blood collection tubes D (two in total), centrifuged for 5 minutes, and 0.85 mL of supernatant was discarded from each of the blood collection tubes. These were combined into one tube, mixed well, poured into the blood collection tube T, stored at room temperature for up to 4 hours in the blood collection tube T, and thereafter stored at 4°C for up to 24 hours. The hematocrit value was measured with a multi-item automatic blood cell analyzer XN-1000 (Sysmex Corporation), and the hematocrit value was 50.1%.

For the blood collection tubes U and V, 5 mL of blood was collected in each of the blood collection tubes D (10 tubes in total), centrifuged for 5 minutes, and 0.85 mL of supernatant was discarded from each of the blood collection tubes. These were combined into one tube and mixed well to prepare a high hematocrit sample. The high hematocrit sample was dispensed in 2 mL portions into a clean polystyrene tube (without additives) to prepare a blood collection tube T, and dispensed in 2 mL portions into a blood collection tube (blood collection tube U) prepared by adding no inosine to the blood collection tube C (for blood glucose) or a blood collection tube (V1 to 5) prepared by adding inosine to the blood collection tube C (for blood glucose) to prepare tubes, and the tubes were stored at room temperature for up to 4 hours and then stored at 4°C for up to 24 hours. The hematocrit value was measured using the blood collection tube T with a multi-item automatic blood cell analyzer XN-1000 (Sysmex Corporation), and the hematocrit value was 50.8%.

### (2) Results

The time-dependent changes in the glucose concentration in the serum and in the glucose concentration in various blood collection tubes at each time are shown in Table 4 below.

**[Table 4]**

| Table 4: Time-dependent change of glucose concentration in various blood collection tubes | | | | | |
|---|---|---|---|---|---|
| Blood collection tube | Inosine Additive amount (mg) | 0 hours (immediately after) (mg/dL) | After 4 hours (mg/dL) | After 12 hours (mg/dL) | After 24 hours (mg/dL) |
| Serum | - | 100 | 101 | 100 | 99 |
| Blood collection tube T | - | 97 | 79 | 63 | 42 |
| Blood collection tube U | - | 98 | 91 | 87 | 87 |
| Blood collection tube V1 | 0.25 | 99 | 93 | 90 | 89 |
| Blood collection tube V2 | 0.50 | 99 | 94 | 92 | 92 |
| Blood collection tube V3 | 0.75 | 98 | 94 | 92 | 92 |
| Blood collection tube V4 | 1.00 | 99 | 95 | 93 | 92 |
| Blood collection tube V5 | 1.25 | 99 | 95 | 93 | 92 |

In the conventional blood collection tube U (blood collection tube C) including sodium fluoride as a glycolysis inhibitor and the blood collection tubes V1 to 5 prepared by adding various concentrations of inosine to the blood collection tube C, the glucose concentration after 24 hours exceeded that of the blood collection tube U, and a high effect of suppressing glucose concentration decrease was observed. Although the hematocrit value of the stored blood was about 50% and the hematocrit value was around the upper limit value of the reference range, the blood collection tube V to which inosine had been added was found to have an effect of suppressing glucose concentration decrease higher than that of the conventional blood collection tube U.

### Example 4: Effect on glucose uptake of red blood cells

### (1) Methods

Blood collected in the blood collection tube B from a volunteer subject was centrifuged at 3000 rpm for 5 minutes within 10 minutes, and plasma was discarded to obtain fresh red blood cells. The red blood cells were washed with PBS(-) and used for the following tests (washed red blood cells).

The test substance was dissolved in the amount described in Table 5 with respect to 1 mL of PBS(-). On the other hand, 5 µL of washed red blood cells immersed in PBS(-) and allowed to stand for about 15 minutes were taken out into a test tube, 150 µL each of a test substance solution was added thereto, and this was heated at 37°C for 15 minutes. Then, 2 µL of a dissolved probe reagent in a glucose uptake assay kit (Glucose Uptake Assay Kit-Green, Dojindo Laboratories Co., Ltd.) was added and heated at 37°C for 45 minutes. The probe reagent was fluorescently labeled glucose (fluorescently labeled glucose), and a powdered reagent dissolved in 20 µL of DMSO was used.

After the immersion in the reagents of the glucose uptake assay was completed, red blood cells were washed 3 times with 150 µL of a cooled 50-fold diluted WI solution (prepared by diluting the solution provided with the assay reagent 50-fold with PBS(-) at 4°C), and 150 µL of a cooled 50-fold diluted WI solution was further added and stirred, and then 20 µL of the solution was dropped onto a glass slide and spread over the slide. Slides were photographed with fluorescence microscope.

The remaining red blood cells were centrifuged to discard the supernatant, and 125 µL of water for injection was added to the remaining lower red blood cells to cause hemolysis. The fluorescence intensity of 100 µL of the supernatant obtained by centrifugation was measured using a microplate reader (MTP-800Lab, Corona Electric Co., Ltd.). The measurement conditions were an excitation light wavelength of 530 nm and a measurement fluorescence wavelength of 492 nm.

### (2) Results

The test substance and the observed fluorescence intensity are shown in Table 5 below.

**[Table 5]**

| Table 5: Fluorescence intensity of fluorescently labeled glucose uptake into red blood cells | | |
|---|---|---|
| Test group | Test substance | Fluorescence intensity |
| 1 | Control (PBS(-)) | 613.5 |
| 2 | Glucose 50 mg/mL | 7.4 |
| 3 | Sodium fluoride 1.5 mg/mL | 255.1 |
| 4 | ATP 1.25 mg/mL | 163.3 |
| 5 | Inosine 2 mg/mL | 6.4 |
| 6 | ATP 2.5 mg/mL + inosine 1 mg + sodium fluoride 1.5 mg/mL | 18.2 |

Test group 1 was in a situation where no test substance was present, and strong fluorescence was observed as a result of uptake of the fluorescently labeled glucose into the red blood cells, leading to accumulation of fluorescence within the red blood cells. In Test group 2, since unlabeled glucose was present in a larger amount than that of fluorescently labeled glucose, competition with the glucose transporter (GLUT1) occurred, and the amount of glucose normally passed was increased, so that the passage of fluorescently labeled glucose was suppressed, and as a result, fluorescence in red blood cells was hardly observed. Test group 5 was in a situation where inosine was present, and as a result of inhibition of the uptake of the fluorescently labeled glucose by inosine, fluorescence in red blood cells was hardly observed as in test group 2. With respect to sodium fluoride and ATP in Test groups 3 and 4, there was a tendency that uptake of fluorescently labeled glucose was suppressed, but the degree of suppression of uptake was much lower than that of inosine. From the above results, it was found that inosine has a strong inhibitory effect on the uptake of glucose in red blood cells.

### Example 5: Influence on test items other than glucose concentration

### (1) Influence on HbA1c measurement

2 mL of whole blood collected from volunteer subjects was stored at room temperature for 24 hours in a conventional blood collection tube (blood collection tube C) containing sodium fluoride and a blood collection tube of the present invention (blood collection tube K) containing 2 mg of inosine and 5 mg of ATP in the blood collection tube, and hemoglobin A1c after storage was measured. As a result, HbA1c was 5.8% in all the blood collection tubes. That is, it was confirmed that the blood collection tube of the present invention did not affect the measurement of HbA1c.

### (2) Effects on insulin and c-peptide measurements

According to the description of (1) of Example 1, blood was collected in a conventional blood collection tube (blood collection tube C) containing sodium fluoride and a blood collection tube of the present invention (blood collection tube K) containing 2 mg of inosine and 5 mg of ATP in the blood collection tube, and the insulin concentration and the c-peptide concentration were measured immediately after blood collection and 24 hours after blood collection. For the conventional blood collection tube and the blood collection tube of the present invention, insulin concentrations in serum and blood collection tube plasma immediately after blood collection are shown in Fig. 3. For the conventional blood collection tube and the blood collection tube of the present invention, c-peptide concentrations in serum and blood collection tube plasma immediately after blood collection are shown in Fig. 4. The insulin concentration and the c-peptide concentration were based on the serum measured value obtained by centrifugation immediately after blood collection, and the measured values of both the conventional blood collection tube and the blood collection tube of the present invention were found to decrease. However, since a decrease in the measured value was observed at a constant rate, it is possible to convert the measured value into a serum measured value by setting a correction coefficient. That is, it was confirmed that the insulin concentration and the c-peptide concentration can be measured by the blood collection tube of the present invention.

## Claims

1. An agent for inhibiting glucose uptake into red blood cells, comprising inosine as an active ingredient.

2. An agent for suppressing glucose concentration decrease in a blood collection tube, comprising inosine as an active ingredient.

3. The agent for suppressing glucose concentration decrease according to claim 2, further comprising a glycolysis inhibitor.

4. The agent for suppressing glucose concentration decrease according to claim 3, wherein the glycolysis inhibitor is one or more selected from the group consisting of a fluoride salt and adenosine phosphate or a salt thereof.

5. The agent for suppressing glucose concentration decrease according to claim 2 or 3, further comprising an anticoagulant.

6. The agent for suppressing glucose concentration decrease according to claim 5, wherein the anticoagulant is one or more selected from the group consisting of ethylenediaminetetraacetic acid (EDTA) and salts thereof, and hydrates thereof, citric acid and a salt thereof, and heparin and a salt thereof.

7. The agent for suppressing glucose concentration decrease according to claim 2 or 3, wherein the inosine is used at 0.10 mg or more per 1 mL of collected blood.

8. A blood collection tube for measuring a glucose concentration in blood, comprising the agent for suppressing glucose concentration decrease according to claim 2 or 3 in an internal space.

9. The blood collection tube according to claim 8, wherein the agent for suppressing glucose concentration decrease contains 0.10 mg or more of inosine per 1 mL of collected blood.

10. The blood collection tube according to claim 8 or 9, for further measuring one or more selected from the group consisting of HbA1c, insulin, and C-peptide.

11. A method for inhibiting glucose uptake into red blood cells in whole blood or a method for suppressing a decrease in a glucose concentration in whole blood, comprising mixing whole blood and inosine in vitro.

12. The method according to claim 11, further comprising mixing a glycolysis inhibitor and/or an anticoagulant with whole blood.

13. The method according to claim 11 or 12, wherein the inosine is mixed in at 0.10 mg or more per 1 mL of whole blood.
